# EUROPÄISCHE PATENTSCHRIFT

(11) **EP 2 091 478 B1**
(45) Veröffentlichungstag und Bekanntmachung des Hinweises auf die Patenterteilung: **28.03.2012**
(21) Anmeldenummer: 07856024.0
(22) Anmeldetag: 23.11.2007
(51) Int. Cl.: A61F 6/00

(54) **KONDOMVERPACKUNG**
CONDOM PACKAGING
EMBALLAGE DE PRÉSERVATIF

(30) Priorität: 24.11.2006 DE 102006056107
(43) Veröffentlichungstag der Anmeldung: 26.08.2009
(73) Patentinhaber: Adamietz, Gerold Achim, 10405 Berlin (DE)
(72) Erfinder: Adamietz, Gerold Achim, 10405 Berlin (DE)
(74) Vertreter: Boeckh, Tobias
(86) Internationale Anmeldenummer: PCT/DE2007/002130
(87) Internationale Veröffentlichungsnummer: WO 2008/061522

(56) Entgegenhaltungen:
- WO-A-92/20595
- WO-A-2004/000682
- DE-A1- 3 237 566
- DE-C- 927 529
- DE-U- 1 781 771
- US-A- 6 076 661

## Beschreibung

Die Erfindung betrifft eine neuartige Kondomverpackung, insbesondere eine anwenderfreundliche anspruchsvolle Verpackung für Kondome.

Im Stand der Technik sind etliche Arten zur Verpackung von Kondomen bzw. Präservativen bekannt. [Einschub Seite 1a]

Die meisten zeichnen sich dadurch aus, dass das Kondom einzeln verpackt in einer Folie verschweißt ist. Dabei erweist sich als Nachteil, dass üblicherweise beim Auspacken eines in Folie verpackten Kondoms nur schwer zu erkennen ist, in welche Richtung das Kondom auszurollen ist; auch kann es dabei leicht zerstört werden, indem beim Aufreißen der Folie das Kondom gleich mit eingerissen wird. Besonders die oft schlechten Lichtverhältnisse bei der Anwendung (z.B. im Dunkeln) tragen zu unangenehmen Pausen bei der Verwendung des Kondoms beim Liebesspiel bei. Das falsche Ansetzen eines Kondoms macht es fernerhin noch zudem unter Umständen unbrauchbar. Die Verwendung eines nachträglich umgedrehten Kondoms macht die Schutzwirkung des Kondoms zunichte. In einem solchen Fall müsste also ein neues Kondom ausgepackt werden, was zu Beeinträchtigungen des Liebesspiels führt und zudem die doppelten Kosten verursacht.

Eine alternative Folienkondomverpackung ist beispielsweise aus der DE 202 15 481 U1 bekannt, die eine Kunststoffschale mit einem aufgewölbten Boden als Verpackung für ein Kondom beschreibt, das unter einer abziehbaren Folie aufgerollt in der Schale liegt. Der Nachteil dieser Kondomverpackung ist darin zu sehen, dass das eigentliche Problem der Erkenn- bzw. Fühlbarkeit der richtigen Ausrichtung des Kondoms auch nicht gelöst wird. Denn von außen ist der Kondomverpackung nicht anzusehen und nicht zu ertasten, wo die Spitze des Kondoms liegt; dies kann allenfalls erst dann geschehen, wenn die Verpackung geöffnet ist. Es bleibt dabei dem bloßen Zufall überlassen, das Kondom richtig zu erfassen und die richtige Ausrichtung des Kondoms zu erkennen bzw. zu ertasten. Im schlimmsten Fall fällt das Kondom einfach aus der Verpackung und ist damit gänzlich unbrauchbar.

So offenbart beispielsweise die US 6 076 661 eine Verpackung für Kondome, die als dünne Plastikkarte gestaltet ist und insbesondere für die Aufnahme von Polyethylen Kondomen geeignet ist. Des Weiteren beschreibt die WO 2004/000682 eine Verpackung, die aus zwei Einheiten besteht und der Aufnahme eines Kondoms und eines Hygienetuches dient. Das Kondom und das Tuch sind in einer zentralen Ausbuchtung in der Verpackung aufbewahrt. Die DE 32 37 566 A1 offenbart eine Verpackung, bei der die Verpackung aus einem Basis- und einem Deckteil bestehen. Das Basisteil hat einen zum Inneren gerichteten Vorsprung, der an dem mittleren Kondomteil anliegt und diesen nach Packungsöffnung in Benutzungsrichtung stützt. Ausgehend von diesem Stand der Technik ist es die Aufgabe der vorliegenden Erfindung, eine Kondomverpackung zu schaffen, die ein sicheres Auspacken des Kondoms aus der Verpackung und eine daraus folgende fehlerfreie Anwendung des Kondoms gewährleistet.

Gelöst wird diese Aufgabe durch die Merkmale der unabhängigen Ansprüche. Erfindungsgemäß ist danach zum einen eine einzelne Kondomverpackung vorgesehen, enthaltend ein Kondom mit Reservoir und Rollrand, umfassend eine Oberseite und eine Unterseite, die lösbar miteinander verbunden sind, dadurch gekennzeichnet, dass die Oberseite korrespondierend zu den Ausmaßen eines zusammengerollten Kondoms einstückig als Korpus ausgeformt ist und insbesondere eine Kavität für die Beherbergung des Rollrandes des Kondoms und eine Kavität für die Beherbergung der die Spitze des Kondoms bildenden Reservoirs aufweist, wobei die Kavitäten aus dem gesamten Korpus soweit herausragen, dass sie einzeln wahrnehmbar ertastbar sind.

Die Kavitäten selbst sind auch optisch erkennbar und sichtbar und ergänzen somit die Ertastbarkeit und damit die korrekte Verwendung der erfindungsgemäßen Kondomverpackung.

Das Kondom selbst lässt sich aus der Kondomverpackung auf einfache Weise herausnehmen, da die Unterseite über den die Oberseite bildenden Korpus hinausragt und insoweit ein Lasche bildet, die über eine Ablöskante von der Oberseite ablösbar ist. Die Unterseite der Kondomverpackung bildet daher eine Art Trägerfolie, die jeweils entsprechend den äußeren Bemassungen der Oberseite / Korpus, in dem ein handelsübliches Kondom beherbergt ist, zugeschnitten ist. Ergänzend ist vorgesehen, dass die gesamte Kondomverpackung oder aber auch nur die Unter- oder die Oberseite aus einem transparenten, opaken und/oder phosphoreszierenden und/oder fluoreszierenden Material gefertigt ist. Dies hat den Vorteil, dass das Kondom im Dunkeln besser erkennbar ist. Besonderes Kennzeichen der Erfindung ist, dass die Außenform des Korpus die Oberseite des innerhalb des Korpus beherbergten Kondoms abbildet.

Erfindungsgemäß ist die sichtbare und ertastbare Wahrnehmung der Kavitäten des Korpus, die den Rollrand bzw. das Reservoir des Kondoms beherbergen, bzw. der dazwischen liegenden konkaven Ausnehmung für die Erkennbarkeit und richtige Orientierung sowie spätere korrekte Positionierung des Kondoms ein wesentliches Merkmal.

Die Ablösung der Oberseite von der Unterseite (bzw. umgekehrt) erfolgt durch Abziehen der die Unterseite bildenden Folie von dem die Oberseite bildenden Korpus. Hierzu weist die Unterseite eine entsprechende Ablöskante auf. Hervorzuheben ist, dass eine Ungleichheit der Steifigkeit der Materialien von Unter- und Oberseite von Vorteil ist. Das Material des die Oberseite bildenden im wesentlichen biegesteifen Korpus ist vorzugsweise steifer ausgebildet ist als die die Unterseite bildende Folie.

Die Erfindung sieht ferner eine Kondomsammelverpackung, bestehend aus mehreren Kondomverpackungen vor, wobei mehrere einzelne Kondomverpackungen, enthaltend jeweils ein Kondom, einstückig als Verbund angeordnet sind, wobei die einzelnen Kondomverpackungen aus dem Verbund der Kondomsammelverpackung ohne Beschädigung des Kondoms herauslösbar sind. Der Vorgang des Herauslösens einer einzelnen Kondomverpackung aus dem Verbund der Kondomsammelverpackung wird über entsprechende Sollbruchstellen an den Verbindungskanten der jeweils weiteren Kondomverpackung des Verbunds der Kondomverpackungen ermöglicht.

Ferner sieht die Erfindung eine Kondomumverpackung vor, umfassend einen Verpackungseinband mit einer Kondomsammelverpackung mit einzelnen Kondomverpackungen, die jeweils ein Kondom beherbergen. Die Kondomumverpackung weist eine Verbindungsfläche auf, mittels derer die Kondomsammelverpackung in der Kondomumverpackung über eine Aufhängeeinrichtung lösbar verbunden ist. Zu Versiegelungszwecken sind auf der auf der Innenseite des Verpackungseinbandes angeordnete Sicherheitselemente vorgesehen, die in unbenutztem Zustand die Unversehrtheit der erfindungsgemäßen Umverpackung anzeigen. Zu Werbezwecken ist ferner vorgesehen, dass die Kondomumverpackung mit einem herauslösbaren und somit abtrennbaren Element verbunden ist, welches vorzugsweise über eine Perforation mit dem Verpackungseinband verbunden ist.

Vorteile der Erfindung lassen sich daher zusammenfassend wie folgt darstellen: Die Lösung des oben beschriebenen Problems besteht darin, eine Verpackung zu schaffen bei der die Lage der Kondomspitze schon vor dem Auspacken bekannt ist.

Die erfindungsgemäße Kondomverpackung, Kondomsammelverpackung und Kondomumverpackung weist folgende Vorteile auf:
- Unverwechselbar ist die Orientierung des verpackten Kondoms schon von außen jederzeit erkennbar.
- Beim sehr bequemen Öffnen der Packung durch eine Lasche liegt das Kondom immer in der selben Richtung.
- Der Konsument kann sowohl die ganze Kondomsammelverpackung mit Papierhülle (also die Kondomumverpackung) benutzen, ebenso ist es möglich, einzelne Kondomverpackungen abzutrennen und einzeln bereitzuhalten.
- Eine Färbung der Trägerfolien erleichtert die Differenzierung innerhalb einer Produktfamilie.
- Die besonders einfache Trennung von Papier und Folienbestandteil der Packung ermöglicht ein besonders einfaches Recycling der verwendeten Wertstoffe und ist dadurch in hohem Maße umweltfreundlich und erfüllt alle gängigen Anforderungen.
- Vorteile für den Anwender sind in folgendem zu sehen: Attraktivität, Zeitersparnis beim Auspacken, Frust- und Risikoverhinderung, keine Verschwendung und daher Kostenersparnis, wesentliche Steigerung der Produktsicherheit.

Die Erfindung lehnt sich dabei in ihrem Erscheinungsbild an die aus der pharmazeutischen Industrie so genannten Blisterpackungen an. In der Arzneimittelbranche werden Blisterpackungen oftmals als Durchdrückpackung verwendet, was allerdings für die vorliegende Erfindung völlig ungeeignet wäre, da das Kondom in diesem Fall aufgrund seiner Flexibilität nicht den nötigen Widerstand bietet, durch die Trägerfolie gedrückt zu werden. Auch besteht die Gefahr, dass das Kondom den Durchdrückvorgang nicht unversehrt übersteht und beispielsweise einreißt. Daher wird erfindungsgemäß nur das Prinzip einer Blisterpackung in veränderter Form für die Verpackung von mehreren Kondomen verwendet. Die erfindungsgemäße Verpackung garantiert eine hygienische, luftdicht verpackte, stabile, bruchsichere und konservierende Aufbewahrung bzw. Bevorratung von Präservativen, die von äußeren Einflüssen wie Luftfeuchtigkeit und Schmutz geschützt ist.

Weitere vorteilhafte Maßnahmen sind in den übrigen Unteransprüchen beschrieben; die Erfindung wird anhand von Ausführungsbeispielen und der nachfolgenden Figuren näher beschrieben; es zeigt:
- Figur 1: zeigt eine erfindungsgemäße Kondomumverpackung im geschlossenen Zustand;
- Figur 2: zeigt eine aufgeklappte erfindungsgemäße Kondomumverpackung mit einzelnen, herausnehmbaren Kondomverpackungen, die in einer Kondomsammelverpackung verpackt bzw. gehalten sind;
- Figur 3a bis 3b: zeigt eine Kondomsammelverpackung, aus der einzelne Kondomverpackungen entfernt sind, in Unterseite (Fig. 3b) und Oberseite (Fig. 3c);
- Figur 4a bis 4c: zeigt einen Entnahmevorgang eines Kondoms aus einer vereinzelten Kondomverpackung;
- Figur 5a und 5c: zeigt in Draufsicht auf die Unterseite und im Schnitt schematisch die Anordnung eines Kondoms in einer erfindungsgemäßen einzelnen Kondomverpackung;
- Figur 6a und 6b: zeigt zur Veranschaulichung die Kondomumverpackung mit der losgelösten Sammelkondomverpackung.

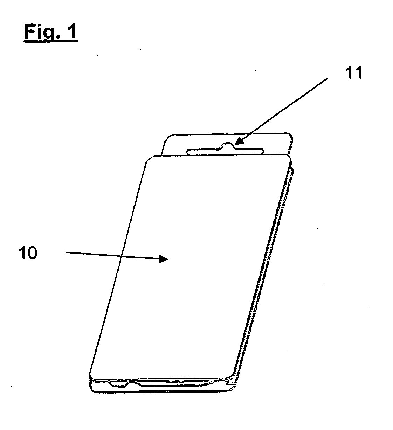

Figur 1 zeigt eine Kondomumverpackung 10 in zugeklapptem Zustand. Aus der Oberkante der Kondomumverpackung 10 ragt eine Aufhängeeinrichtung 11 heraus, die Teil einer Kondomsammelverpackung 13 ist. Diese Aufhängeeinrichtung 11 dient zweierlei Zwecken: Zum einen kann die gesamte Kondomumverpackung 10 über die Aufhängeeinrichtung 11 in einen Präsentationsständer eingehängt werden, zum anderen dient sie als Festhalteelement der Kondomsammelverpackung 13, wenn einzelne Kondomverpackungen 18 aus dem Verbund der Kondomsammelverpackung abgetrennt werden.

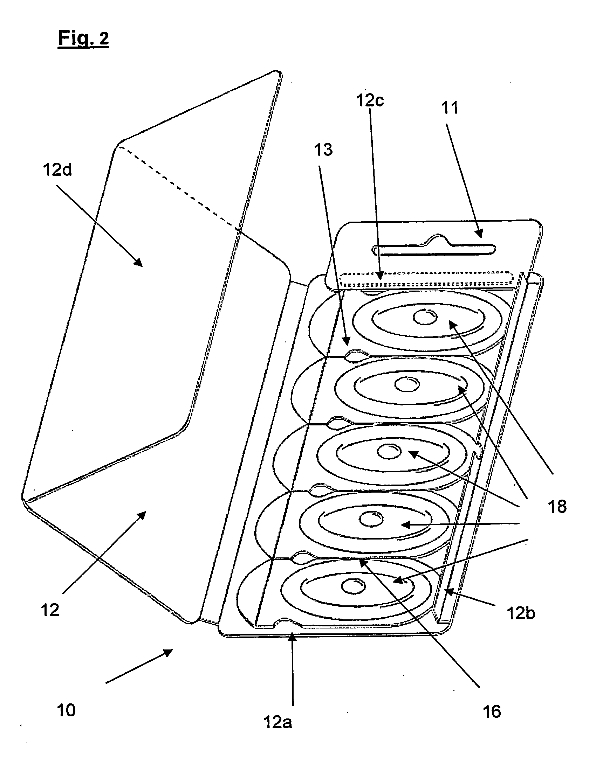

Figur 2 zeigt die Kondomumverpackung 10 in aufgeklapptem Zustand. Die Kondomumverpackung 10, umfassend einen Verpackungseinband 12 kann aus nahezu jedem beliebigen Material bestehen, also beispielsweise aus Pappe, Papier oder einem Kunststoff, die werbeüblich eingefärbt sein können. Der Verpackungseinband 12 dient gleichzeitig als Werbeträger und zur Aufnahme von Gebrauchsanweisungen in schriftlicher Form. Auf der Innenseite 12a der Kondomumverpackung 10 ist eine Kondomsammelverpackung 13 angeordnet, die über eine Verbindungsfläche 12c, die wiederum am Kopfteil der Innenseite 12a angeordnet ist, die Kondomsammelverpackung 13 hält. Bei dieser Verbindungsfläche 12c kann es sich beispielsweise um ein Doppelklebeband oder einen Klettverschluss oder auch einzelne linear angeordnete Haltepunkte handeln. Mit der Verbindungsfläche 12c wird die Aufhängeeinrichtung 11 der Kondomsammelverpackung 13 verbunden. Diese Verbindung kann durch Verkleben oder Verpressen erfolgen, wobei andere Befestigungstechniken durchaus denkbar sind; dabei kommt es jedoch darauf an, dass die Verbindung derartig stark ausgebildet ist, dass Sie sich nicht ablöst, wenn aus dem Verbund der Kondomsammelverpackung 13 eine einzelne Kondomverpackung 18 abgetrennt wird. Die Aufhängeeinrichtung 11 dient daher auch als Festhalteelement. Daneben dient sie der Präsentation der gesamten Kondomumverpackung in einem Verkaufs-oder Präsentationsständer und weist dazu eine Einhängeöffnung auf. Die Verbindung zwischen Verbindungsfläche 12c und Aufhängeeinrichtung 11 der Kondomsammelverpackung 13 soll jedoch auch wieder voneinander lösbar sein, damit zu Entsorgungs- und Recyclingzwecken der Kunststoffanteil gegebenenfalls von dem Papieranteil zu trennen ist.

Daneben weist der Verpackungseinband 12 auf der Innenseite 12a angeordnete Versiegelungs- und Sicherheitselemente 12b (beispielsweise aus Pappe oder Kunststoff) auf, mit denen im Rahmen des Herstellungsprozesses die Kondomumverpackung verschlossen wird und die erst von dem Benutzer dann (durch Aufreißen bzw. Auftrennen) geöffnet werden, so dass dem Benutzer dadurch die Unversehrtheit und eine Erstöffnungsgarantie für die in der Verpackung befindlichen Kondomverpackungen signalisiert wird.

Ferner kann die Kondomumverpackung 10 mit einem herauslösbaren und somit abtrennbaren Element 12d verbunden sein, welches vorzugsweise über eine Perforation mit dem Verpackungseinband 12 verbunden ist. Ein solches Element 12d kann beispielsweise als Werbemittel, Werbeträger, Postkarte, Gutschein, Bestellkarte, Sammelbild oder anderweitig kundenbindendes Element ausgestaltet sein.

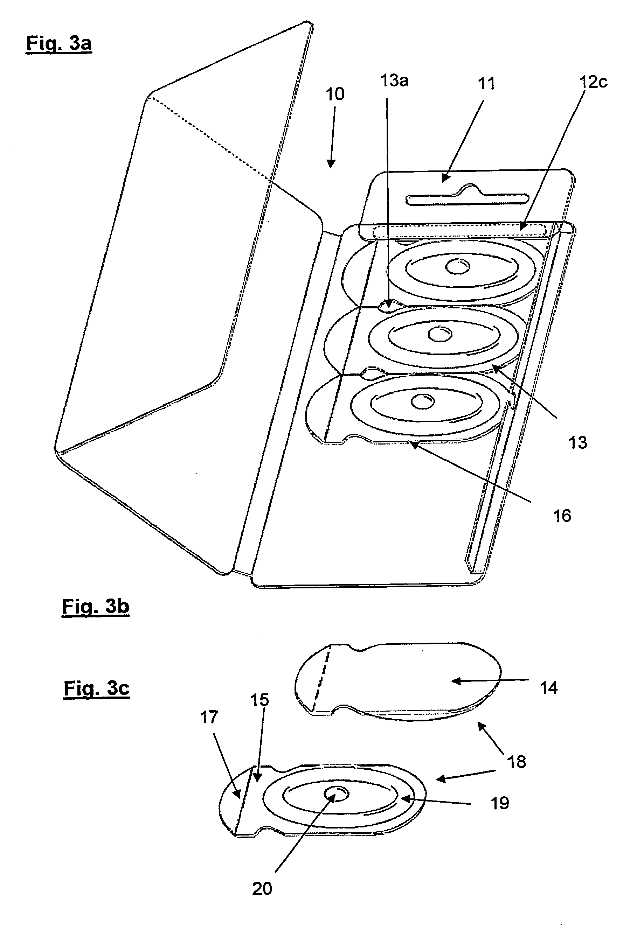

Figur 3a zeigt eine Kondomsammelverpackung 13, aus der einzelne Kondomverpackungen 18 bereits entfernt sind. Damit eine einzelne Kondomverpackung 18 aus dem Verbund einer Kondomsammelverpackung 13 entfernbar ist, weist die Kondomsammelverpackung 13 jeweils - z.B. vorperforierte - Sollbruchstellen 16 an den Verbindungskanten der einzelnen Kondomverpackungen 18 auf, wobei auch eine Sollbruchstelle 16 zur Aufhängevorrichtung 18 vorgesehen ist. Die einzelnen Kondomverpackungen sind mithin auf einfache Weise abknickbar und so aus dem Verbund lösbar, ohne dass die übrigen Kondomverpackungen in Mitleidenschaft gezogen werden, insbesondere nicht beschädigt werden. Eine Aussparung 13a innerhalb der Sollbruchstelle 16 dient als Trennhilfe.

Die Komdomverpackung selbst, insbesondere deren Oberteil 15, welches den dreidimensionalen, einstückigen Korpus zur Aufnahme und Beherbergung des Kondoms bildet, besteht dabei vorzugsweise aus einem geeigneten Kunststoff, der zum einen eine hohe Steifigkeit und Bruchsicherheit, aber auch genügend Flexibilität besitzt, damit die einzelnen Kondomverpackungen aus dem Verbund der Kondomsammelverpackung abtrennbar sind. Das für die Oberseite, aber auch für die Unterseite verwendete Material kann transparent, opak und/oder phosphoreszierend und/oder fluoreszierend sein. Die Unterseite 14, die die Oberseite 15 luft- und wasserdicht abschließt und verschließt, ist vorzugsweise als biegsame Folie ausgestaltet, die von dem die Oberseite 15 bildenden im wesentlichen biegesteifen Korpus abziehbar ist. Der die Oberseite 15 bildende im wesentlichen biegesteife Korpus sollte zumindest steifer ausgebildet sein als die die Unterseite 14 bildende Folie. Die Trägerfolie ist mit dem die Oberseite 15 bildende Korpus verschweißt oder verklebt.

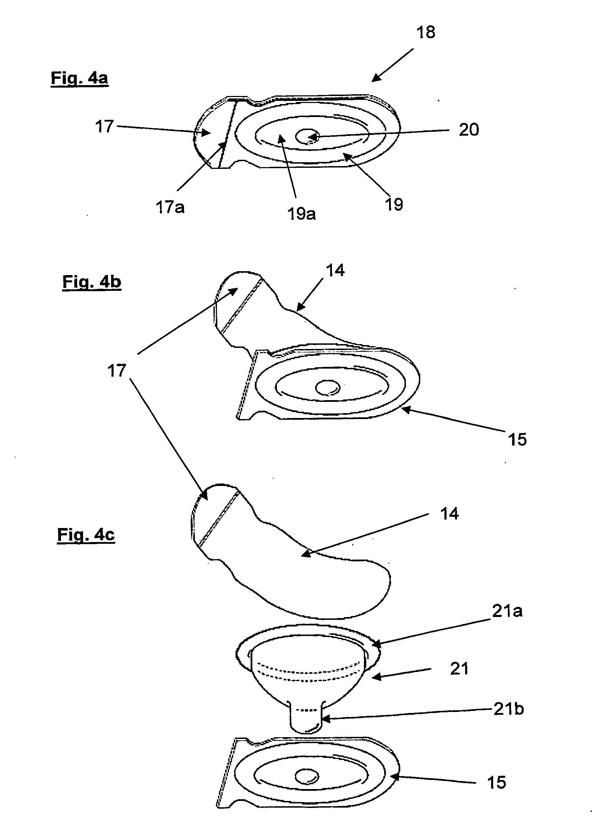

Die Figuren 3b und 3c zeigen eine vereinzelte Kondomverpackung 18 mit Blick auch die Unterseite 14 (Fig. 3b) und auf die Oberseite (Fig. 3c). Die Unterseite 14 der Kondomverpackung ist flächig ausgebildet und bündig mit der Oberseite 15 verbunden, beispielsweise verschweißt. Wie näher in den Figuren 4a bis 4c gezeigt, lässt sich die Unterseite 14 von der Oberseite 15 leicht abziehen; hierzu weist die Unterseite eine Lasche 17 auf, die über den die Oberseite 15 bildenden Korpus hinausragt und über eine Ablöskante 17a, die einer Sollbruchstelle im Korpus entspricht, mit demselben verbunden ist. Die Ablöskante kann jedoch auch an einem beliebigen anderen Ort der Kondomverpackung angeordnet sein, beispielsweise als diagonal verlaufende Ablöskante über die gesamte Unterseite 14 oder als waagrechte oder senkrechte, die Ellipse der Oberseite 15 teilende Ablöskante. Hierbei muss berücksichtigt werden, dass eine bevorzugte Ausformung der Kondomverpackung 13 eine ellipsoide Verpackungsform darstellt; denn ein solche ist zum einen platzsparend, zum anderen verleiht diese Art der Formgebung dem die Oberseite 15 bildenden Korpus eine besondere Stabilität und verhindert beispielsweise ein Verknicken oder Zusammenstauchen der Verpackung und damit letztlich des Kondoms, was schließlich zur Unbrauchbarkeit desselben führen könnte. Denn insbesondere die Rollrandkavität 20 verhindert ein Verknicken oder Zusammenstauchen, da dies über den Widerstand der Rollrandkavität der Kondomverpackung 13 geschehen müsste.

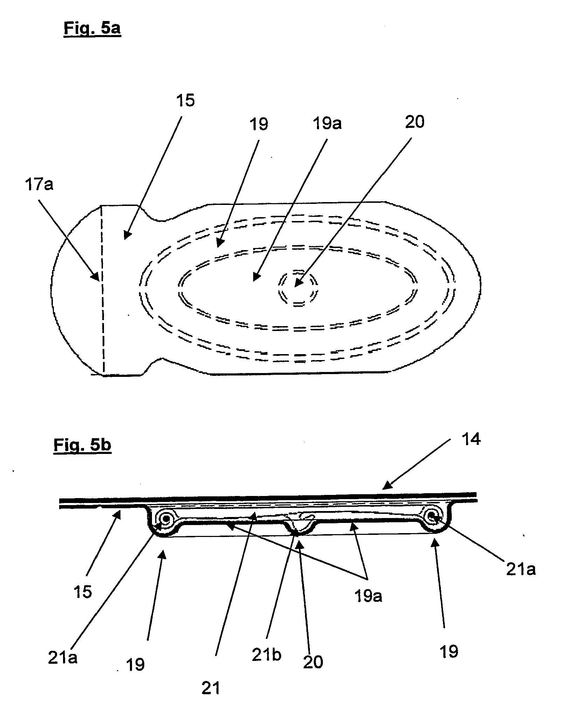

Die Figuren 5a und 5 c zeigen in Draufsicht auf die Unterseite (Fig. 5a) und als Schnittzeichnung (Fig. 5b) schematisch die Anordnung eines Kondoms 21 in einer erfindungsgemäßen einzelnen Kondomverpackung 18. Ein Kondom in zusammengerollten Zustand weist als besondere Merkmale den Rollrand 21 a auf; der Rollrand wiederum besteht aus einem Gummiring, auf dem das Kondom der gesamten Länge nach aufgerollt ist und über den das Kondom bestimmungsgemäß über den Penis abrollbar ist. Daneben weisen die üblichen Präservative an ihrer Spitze ein so genanntes Reservoir 21 b zur Aufnahme der Samenflüssigkeit auf. Die erfindungsgemäße Kondomverpackung 18 weist entsprechend in dem Oberseitenkorpus 15 Kavitäten 19 und 20 auf, d.h. passgenaue Aufnahmen für den Rollrand des Kondoms (19, 21 a) und mittig eine Kavität für das Reservoir des Kondoms (20, 21b). Diese Formgebung führt dazu, dass die Außenform des Korpus der Oberseite 15 den Inhalt, nämlich das Kondom mit Rollrand und Spitze, weitestgehend genau abbildet (sogenanntes logisches Abbild). Diese Formgebung führt gleichsam auch dazu, dass in den Zwischenräumen der Kavitäten eine konkave Fläche 19a ausgeformt wird, die ebenfalls ertastbar ist.

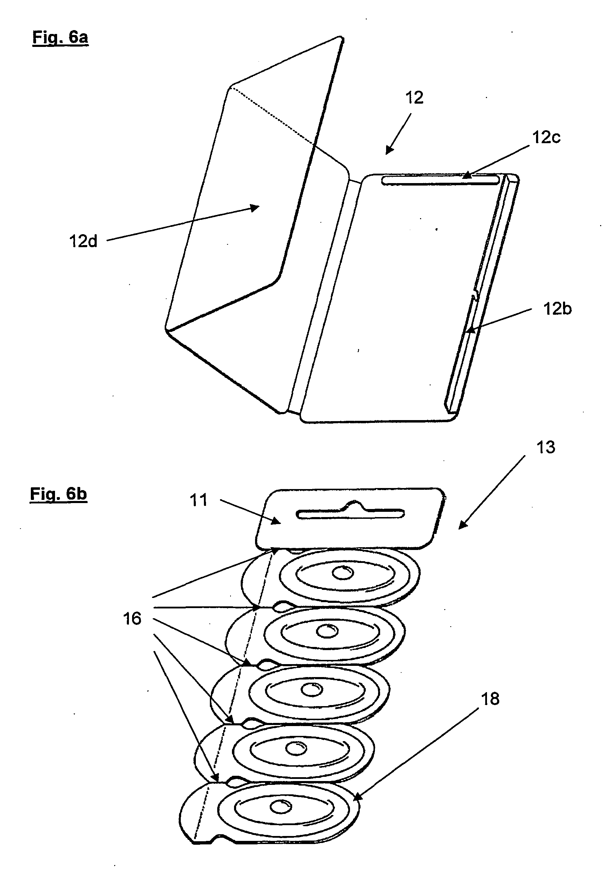

Die Figuren 6 a und 6b zeigen zur Veranschaulichung nochmals die Kondomumverpackung 10, insbesondere den Verpackungseinband 12 mit der im Kopfteil angeordneten Verbindungsfläche 12c zur Verbindung mit der Kondomsammelverpackung 13, insbesondere der dortigen Aufhängeeinrichtung 11, die mit der Verbindungsfläche 12c verklebt oder verschweißt wird.

### Bezugszeichenliste

- 10: Kondomumverpackung
- 11: Aufhängeeeinrichtung
- 12: Verpackungseinband
- 12a: Verpackungseinband / Innenseite
- 12b: Versiegelung / Sicherheiteselement
- 12c: Verbindungsfläche
- 12d: abtrennbares Element des Verpackungseinbands 12
- 13: Kondomsammelverpackung mit einzelnen Kondomverpackungen
- 13a: Aussparung
- 14: Rückseite / Unterseite einer vereinzelten Kondomverpackung
- 15: Korpus / Oberseite einer vereinzelten Kondomverpackung
- 16: Sollbruchstelle
- 17: Lasche
- 17a: Ablöskante / Sollbruchstelle
- 18: Einzelne Kondomverpackung eines Kondoms
- 19: Kavität für Rollrand 21 a
- 19a: konkave Ausnehmung zwischen den Kavitäten
- 20: Kavität für Reservoir 21 b
- 21: Kondom
- 21a: Rollrand des Kondoms
- 21 b: Reservoir des Kondoms

## Patentansprüche

1. Kondomverpackung, enthaltend ein Kondom mit Reservoir und Rollrand, umfassend eine Oberseite und eine Unterseite, die lösbar miteinander verbunden sind wobei die Oberseite (15) korrespondierend zu den Ausmaßen eines zusammengerollten Kondoms (21) einstückig als Korpus ausgeformt ist, **dadurch gekennzeichnet, dass** die Oberseite (15) eine Kavität (19) für die Beherbergung des Rollrandes (21a) des Kondoms (21) und eine Kavität (20) für die Beherbergung der die Spitze des Kondoms (20) bildenden Reservoirs (21 b) aufweist, wobei die Kavitäten (19, 20) aus dem gesamten Korpus (15) soweit herausragen, dass sie einzeln wahrnehmbar ertastbar sind.

2. Kondomverpackung nach Anspruch 1, **dadurch gekennzeichnet, dass** die Außenform des Korpus der Oberseite (15) das innerhalb des Korpus beherbergten Kondom abbildet.

3. Kondomverpackung nach Anspruch 1 oder 2, **dadurch gekennzeichnet, dass** diese eine ellipsoide Gestaltung aufweist.

4. Kondomverpackung nach wenigstens einem der vorhergehenden Ansprüche, **dadurch gekennzeichnet, dass** zwischen den Kavitäten (19, 20) eine konkave Zwischenfläche (19a) ausgeformt ist.

5. Kondomverpackung nach wenigstens einem der vorhergehenden Ansprüche 1 bis 4, **dadurch gekennzeichnet, dass** die Unterseite (14) über den die Oberseite (15) bildenden Korpus hinausragt und insoweit ein Lasche (17) bildet, die über eine Ablöskante (17a) von der Oberseite (15) ablösbar ist.

6. Kondomverpackung nach wenigstens einem der vorhergehenden Ansprüche 1 bis 4, **dadurch gekennzeichnet, dass** die Ablöskante (17a) an einem beliebigen Ort der Unterseite (14) angeordnet ist.

7. Kondomverpackung nach wenigstens einem der vorhergehenden Ansprüche, **dadurch gekennzeichnet, dass** die Oberseite (15) und/oder Unterseite (14) aus einem transparenten, opaken, phosphoreszierenden und/oder fluoreszierenden Material gefertigt ist.

8. Kondomverpackung nach wenigstens einem der vorhergehenden Ansprüche, wobei die Unterseite (14) als Trägerfolie für die in den Ausmaßen eines Kondoms vorgeformte Oberseite (15) dient.

9. Kondomverpackung nach wenigstens einem der vorhergehenden Ansprüche, wobei das Material des die Oberseite (15) bildenden im wesentlichen biegesteifen Korpus steifer ausgebildet ist als die die Unterseite (14) bildende Folie.

10. Kondomsammelverpackung, bestehend aus mehreren Kondomverpackungen gemäß den Ansprüchen 1 bis 9, **dadurch gekennzeichnet, dass** mehrere einzelne Kondomverpackungen (18), enthaltend jeweils ein Kondom (21), einstückig als Verbund angeordnet sind, wobei die einzelnen Kondomverpackungen (18) aus dem Verbund der Kondomsammelverpackung (13) ohne Beschädigung des Kondoms (21) herauslösbar sind.

11. Kondomsammelverpackung nach Anspruch 10, **dadurch gekennzeichnet, dass** zur Herauslösung einer Kondomverpackung (18) aus dem Verbund der Kondomsammelverpackung (13) die einzelnen Kondomverpackungen (18) entsprechende Sollbruchstellen (16) an ihren Verbindungskanten mit der jeweils weiteren Kondomverpackung (18) des Verbunds der Kondomverpackungen (18) und zur Aufhängevorrichtung (11) aufweisen.

12. Kondomsammelverpackung nach Anspruch 10 oder 11, **dadurch gekennzeichnet, dass** diese eine Aufhängeeinrichtung (11) zur Befestigung innerhalb einer Kondomumverpackung (10) aufweist.

13. Kondomsammelverpackung nach Anspruch 12, **dadurch gekennzeichnet, dass** die Aufhängevorrichtung Teil der Kondomsammelverpackung (13) ist.

14. Kondomumverpackung, umfassend einen Verpackungseinband (12) mit einer Kondomsammelverpackung nach Anspruch 10 bis 13, wobei einzelne Kondomverpackungen (18) über Sollbruchstellen (16) aus dem Verbund der Kondomsammelverpackung (13) entnehmbar sind..

15. Kondomumverpackung nach Anspruch 14, wobei die Kondomsammelverpackung (13) eine Verbindungsfläche (12c) aufweist, mittels derer die Kondomsammelverpackung (13) in der Kondomumverpackung (10) über eine Aufhängeeinrichtung (11) lösbar verbunden ist und wobei der Verpackungseinband (12) auf der Innenseite (12a) angeordnete Versiegelungs- und Sicherheitselemente (12b) aufweist.

## Claims

1. Condom package containing a condom with reservoir and rolled edge consisting of a top side and a lower side which are connected and separable. The top side (15) is a one-piece corpus formed according to the dimensions of a rolled up condom (21); the top side (15) features a cavity (19) for accommodating the rolled edge (21a) of a condom (21) and a cavity (20) for accommodating the reservoir (21b) forming the top of the condom (20). The cavities (19, 20) project so far out of the entire corpus (15) that there shape can be individually felt.

2. Condom package according to claim 1, **characterized in that** the outside shape of the corpus of the top side (15) represents the condom accommodated within the corpus.

3. Condom package according to claim 1 or 2, **characterized in that** it has an ellipsoid shape.

4. Condom package according to at least one of the previous claims, **characterized in that** a concave intermediate surface (19a) is formed between the cavities (19, 20).

5. Condom package according to at least one of the previous claims 1 to 4, **characterized in that** the lower side (14) extends beyond the corpus making up the top side (15) and that it forms a flap (17), which can be separated from the top side (15) along the separating edge (17a).

6. Condom package according to at least one of the previous claims 1 to 4, **characterized in that** the separating edge (17a) is located anywhere on the lower side (14).

7. Condom package according to at least one of the previous claims, **characterized in that** the top side (15) and/or the lower side (14) is made of a transparent, opaque, phosphorescent and/or fluorescent material.

8. Condom package according to at least one of the previous claims whereas the lower side (14) serves as the carrier foil for the top side (15) which is preformed according to the size of a condom.

9. Condom package according to at least one of the previous claims with the material of the essentially flexural resistant corpus that makes up the top side (15) is stiffer than that of the foil that makes up the lower side (14).

10. Condom multi-pack consisting of several condom packages according to the claims 1 to 9, **characterized in that** several individual condom packages (18) - containing one condom (21) each - make up a one piece assemblage. The individual condom packages (18) can be removed from the condom multi-pack (13) without damaging the condom (21).

11. Condom multi-pack according to claim 10, **characterized in that** for the removal of a condom package (18) from the assemblage of the condom multi-pack (13), the individual condom packages (18) have corresponding breaking points (16) located along their joining edges that connect them to the adj acent condom package (18) of the condom package assemblage (18) and the hanging device (11).

12. Condom multi-pack according to claim 10 or 11, **characterized in that** it features a hanging device (11) for attaching it inside an outer condom package (10).

13. Condom multi-pack according to claim 12, **characterized in that** the hanging device is part of the condom multi-pack (13).

14. Outer condom package consisting of a package cover (12) with a condom multi-pack according to claim 10 to 13. Individual condom packages (18) can be removed via breaking points (16) from the assemblage of the condom multi-pack (13).

15. Outer condom package according to claim 14 in which the condom multi-pack (13) features a joining surface (12c), which connects the condom multi-pack (13) to the outer condom package (10) by means of a hanging device (11) allowing them to be separated . The package cover (12) features sealing and safety elements (12b) on the inside (12a).

## Revendications

1. Emballage de préservatif contenant un préservatif avec réservoir et bord déroulant, comprenant une partie supérieure et une partie inférieure associées entre elles de façon détachable, la partie supérieure (15) étant formée pour correspondre aux dimensions d'un préservatif enroulé (21) en une seule unité comme corps, la partie supérieure (15) présentant une cavité (19) pour le logement du bord déroulant (21a) du préservatif (21) et une cavité (20) pour le logement du réservoir (21b) formant la pointe du préservatif (20), les cavités (19, 20) dépassant de l'ensemble du corps (15) de façon qu'elles soient perceptiblement reconnaissables individuellement au toucher.

2. Emballage de préservatif selon la revendication 1 **caractérisé en ce que** la forme extérieure du corps de la partie supérieure (15) reproduit le préservatif logé dans le corps.

3. Emballage de préservatif selon la revendication 1 ou 2 **caractérisé en ce que** celui-ci présente une forme ellipsoïdale.

4. Emballage de préservatif selon au moins l'une des revendications précédentes **caractérisé en ce qu'**entre les cavités (19, 20), une surface (19a) concave est formée.

5. Emballage de préservatif selon au moins l'une des revendications précédentes 1 à 4 **caractérisé en ce que** la partie inférieure (14) dépasse au-dessus du corps formant la partie supérieure (15) et forme ainsi une languette (17) pouvant être détachée de la partie supérieure (15) par un bord de détachement (17a).

6. Emballage de préservatif selon au moins l'une des revendications précédentes 1 à 4 **caractérisé en ce que** le bord de détachement (17a) est placé à un endroit quelconque de la partie inférieure (14).

7. Emballage de préservatif selon au moins l'une des revendications précédentes **caractérisé en ce que** la partie supérieure (15) et/ou la partie inférieure (14) est/sont fabriquée/s à partir d'un matériau transparent, opaque, phosphorescent et/ou fluorescent.

8. Emballage de préservatif selon au moins l'une des revendications précédentes, la partie inférieure (14) servant de film support pour la partie supérieure (15) préformées dans les dimensions d'un préservatif.

9. Emballage de préservatif selon au moins l'une des revendications précédentes, le matériau du corps formant la partie supérieure (15) essentiellement rigide à la flexion étant formée de façon plus rigide que le film formant la partie inférieure (14).

10. Emballage groupé de préservatifs composé de plusieurs emballages de préservatif composés selon les revendications 1 à 9 **caractérisé en ce que** plusieurs emballages de préservatif individuel (18), contenant respectivement un préservatif (21), sont placés en une unité comme ensemble, les différents emballages de préservatif (18) pouvant se détachés de l'ensemble de l'emballage groupé de préservatif (13) sans endommager le préservatif (21).

11. Emballage groupé de préservatifs selon la revendication 10 **caractérisé en ce que** pour détacher un emballage de préservatif (18) hors de l'ensemble de l'emballage groupé de préservatif (13), les différents emballages de préservatif (18) présentent des points destinés à la rupture (16) à leurs bords de jonction avec l'autre emballage de préservatif (18) de l'ensemble des emballages de préservatif (18) et vers l'élément d'accrochage (11).

12. Emballage groupé de préservatifs selon la revendication 10 ou 11 **caractérisé en ce que** celui-ci présente un élément d'accrochage (11) pour fixation dans le suremballage de préservatifs (10).

13. Emballage groupé de préservatifs selon la revendication 12 **caractérisé en ce que** l'élément d'accrochage est une partie de l'emballage groupé de préservatif (13).

14. Suremballage de préservatifs composé d'une couverture (12) avec un emballage groupé de préservatifs selon la revendication 10 à 13, les différents emballages de préservatifs (18) pouvant être retirés de l'ensemble de l'emballage groupé de préservatifs (13) grâce aux points destinés à la rupture (16).

15. Suremballage de préservatifs selon la revendication 14 **caractérisé en ce que** l'emballage groupé de préservatifs (13) présente une surface de jonction (12c) à l'aide de laquelle l'emballage groupé de préservatifs (13) dans le suremballage (10) est attaché de façon détachable via un suremballage (11) et la couverture d'emballage (12) présentant des éléments de scellement et de sécurité (12b) disposés sur le côté intérieur (12a).
